# EUROPEAN PATENT APPLICATION

(11) **EP 2 959 822 A1**
(43) Date of publication of application: **30.12.2015**
(21) Application number: 14754666.7
(22) Date of filing: 20.02.2014
(51) Int. Cl.: A61B 1/00, A61F 2/82, A61F 2/958, A61M 25/10, A61M 31/00, A61M 37/00

(54) **MEANS FOR CONTROLLED RELEASE OF FLUID, AND ENDOSCOPE AND ENDOSCOPIC SURGICAL INSTRUMENT COMPRISING SAME**

(30) Priority: 21.02.2013 JP 2013032498
(71) Applicant: Olympus Corporation, Shibuya-ku Tokyo 151-0072 (JP)
(72) Inventor: IGA Yasunobu, Tokyo 151-0072 (JP); TANIKAWA Yohei, Tokyo 151-0072 (JP); TAKIMOTO Shinichi, Tokyo 151-0072 (JP)
(74) Representative: Schicker, Silvia
(86) International application number: PCT/JP2014/054059
(87) International publication number: WO 2014/129552

(57) **Abstract**

The present invention offers a means for sustained release of liquid making it possible to continue to stably and efficiently administer liquid like medical solution also to a diseased region which exists on a luminal wall surface against gravity, making it possible to easily move the means after administration of liquid, making it possible to optionally adjust an amount of administration of the liquid and administration rate of the liquid, and making it possible to administer plural kinds of liquid in turn; an endoscope having the same; and an instrument for endoscopic surgery having the same.

An means for sustained release of liquid according to the present invention includes: an outer pouch-shaped elastic member 2; an inner pouch-shaped elastic member placed inside the outer pouch-shaped elastic member and capable of being inflated; a liquid-holding section 3 holding liquid between the inner pouch-shaped elastic member and the outer pouch-shaped elastic member; and minute holes provided for the outer pouch-shaped elastic member, the diameter of the minute holes being changed by inflation and deflation.

## Description

### Technical Field

This invention relates to: a means for sustained release of liquid which is used for giving liquid like medical solution to a diseased region on a luminal wall surface of a living body for example; an endoscope having the same; and an instrument for endoscopic surgery having the same.

### Background Art

Conventional problems in endoscopic treatments which ESD (Endoscopic submucosal dissection) represents include intraoperative bleeding and postoperative bleeding.

However, an optical clearing agent capable of improving living-body permeability of light by suppressing light scattering has been developed in recent years.

Optical clearing agent is an aqueous solution acquired by adding glycerol and surfactant to urea, and the aqueous solution is given to a living tissue to penetrate into the living tissue and then makes a penetrated region of the living tissue transparent like a jelly. Besides, the region that is made transparent by the optical clearing agent can be returned into its original state in which the penetrated region was opaque, again, by giving saline or the like to the penetrated part.

In order to reduce the risks of intraoperative bleeding and postoperative bleeding, it is desired that an optical clearing agent is given to a diseased part before or after surgery so that vascular visibility is improved.

By the way, it is characteristic of such an optical clearing agent to take predetermined time to penetrate into a living tissue that is given the optical clearing agent. As a result, it is impossible to observe a target region immediately after the target region is given the optical clearing agent. Accordingly, a use of optical clearing agent in surgery requires a means for giving optical clearing agent in advance before excision of a part to be excised.

However, a diseased region to which an optical clearing agent should be applied does not necessarily exist in the gravity direction in a lumen. As a result, the optical clearing agent is not easy to give to a diseased region existing in the opposite direction to the gravity direction while the optical clearing agent is being kept stably remaining at the diseased region.

In conventional techniques for giving an agent to a diseased region with the agent keeping stably remaining at the diseased region, the inventions disclosed in the following Patent Literatures 1 and 2 respectively have been proposed for example, the Patent Literature 1 disclosing a drug sustained-release stent in which a stent is covered with polymer containing agent, and the Patent Literature 2 disclosing an agent-applying instrument which is aimed at applying anesthetic to nasal cavity.

### Prior Art Literature List

### Patent Literature

Patent Literature 1: WO09 / 031295
Patent Literature 2: Japanese Patent Kokai No. 2008-188212

### Summary of Invention

### Technical Problem

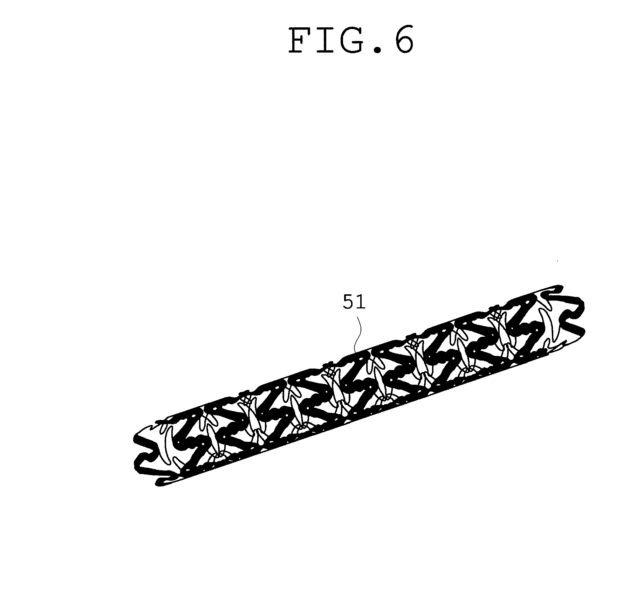

As shown in Fig. 6, the drug sustained-release stent disclosed in Patent Literature 1 includes: a cylinder-shaped stent body 51 having an outer surface and an inner surface; the first cover layer covering at least the outer surface of the stent body 51; and the second cover layer substantially completely covering the first cover layer. Each of the first and second cover layers contains a predetermined agent which is molecularly dispersed or is micro-dispersed with the agent in a fine solid state, in a polymer tissue (which is: a compact tissue in which no void is observed through optical microscope observation; porous tissue; or the like). And, the drug sustained-release stent disclosed in Patent Literature 1 is configured to supply an agent while a target region like a narrowed region is being kept expanded by the stent inserted into the target region.

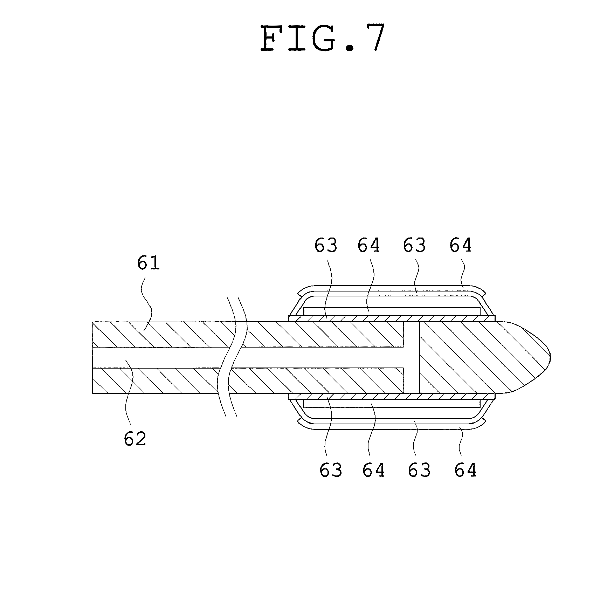

Also, as shown in Fig. 7, the agent-applying instrument disclosed in Patent literature 2 includes: an elongated stick-shaped base components 61; a gas passage 62 running along the base component; a cylinder-shaped balloon 63 placed on the whole circumference of the outside surface of the base component 61 and communicating with the gas passage 62; and an agent-holding component 64 placed on the outside surface of the balloon 63 and containing an agent that is applied to a body cavity. And, the agent-applying instrument disclosed in Patent literature 2 is configured to apply an anesthetic to a target region by supplying air to the gas passage 62 with a gas-supplying instrument to inflate the balloon 63 so that the agent-holding component 64 comes into contact with the target region to press the inside surface of the target region.

However, in the case where the stent disclosed in Patent Literature 1 is used as a means for administering optical clearing agent, users have trouble in retrieving the stent after a diseased region is made transparent, which inevitably hinders diagnosis of or treatment of the diseased region having been made transparent. Also, when the optical clearing agent is supplied through: the first cover layer covering the outer surface of the stent body 51; and the second cover layer as in Patent Literature 1, it is hard to optionally regulate an amount of administration of the optical clearing agent or a rate at which the optical clearing agent is supplied. In addition, it is difficult to give another kind of liquid like saline for returning the diseased region to its original state in which the diseased region is opaque, after administration of the optical clearing agent.

Also, in the agent-applying instrument disclosed in Patent Literature 2, the agent-holding component 64 is configured to be: a fibrous member having moisture-retaining property; or a concave portion holding agent so that the agent-holding component 64 holds the agent. However, in the case where the optical clearing agent is supplied through: a fibrous member having moisture-retaining property; or a concave portion holding the agent, it is hard to optionally regulate an amount of administration of optical clearing agent or a rate at which the optical clearing agent is supplied.

Also, Patent Literature 2 suggests that an agent-applying instrument includes an agent passage for supplying additional amount of agent to the agent-holding component 64 (the agent passage being omitted in the drawings). However, the agent passage in the agent-applying instrument disclosed in Patent Literature 2 is exposed to the outside in the vicinity of the agent-holding component 64 on the surface of the base component 61 and does not connect with the agent-holding component 64. As a result, in the case where the agent-applying instrument is configured to include the agent passage as disclosed in Patent Literature 2, liquid like optical clearing agent inevitably flows down on the surface of the base component 61 in supply of additional amount of the liquid to the agent-holding component 64 when a target region for administration of agent exists in the direction opposite to the gravity direction, so that it is difficult to efficiently supply the liquid to the agent-holding component 64. In addition, it is hard to optionally regulate an amount of administration of optical clearing agent or a rate at which the optical clearing agent is supplied.

The present invention is made in view of such conventional problems. The objective of the present invention is to offer: a user-friendly means for sustained release of optical clearing agent which makes it possible to continue to stably and efficiently supply liquid like medical solution also to a diseased region existing against gravity on the luminal wall surface, which makes it possible to easily change a position of the means after administration of the liquid, which makes it possible to optionally regulate an amount of administration of the liquid or a rate at which the liquid is supplied, and which makes it possible to supply plural kinds of liquid in turn; an endoscope having the same; and an instrument for endoscopic surgery having the same.

### Solution to Problem

In order to achieve the above objective, a means for sustained release of liquid according to the present invention is characterized in that the means includes: an outer pouch-shaped elastic member; an inner pouch-shaped elastic member which is placed inside the outer pouch-shaped elastic member and which can be inflated; a liquid-holding section which holds liquid between the inner pouch-shaped elastic member and the outer pouch-shaped elastic member; and a minute hole which is provided for the outer pouch-shaped elastic member and the diameter of which is changed by inflation and deflation.

Also, it is preferred that a means for sustained release of liquid according to the present invention further includes a liquid-infusing section which can infuse liquid into the liquid-holding section between the inner pouch-shaped elastic member and the outer pouch-shaped elastic member.

Also, in a means for sustained release of liquid according to the present invention, it is preferred that a group of minute holes is provided for only a partial area of the outer pouch-shaped elastic member.

Also, in a means for sustained release of liquid according to the present invention, it is preferred that the partial area of the outer pouch-shaped elastic member is an area which forms the liquid-holding section in the outer pouch-shaped elastic member.

Also, in a means for sustained release of liquid according to the present invention, it is preferred that the elasticity of an area of the outer pouch-shaped elastic member excluding an area of the outer pouch-shaped elastic member forming the liquid-holding section is lower than that of the area of the outer pouch-shaped elastic member forming the liquid-holding section.

Also, in a means for sustained release of liquid according to the present invention, it is preferred that the elasticity of an area of the inner pouch-shaped elastic member excluding an area of the inner pouch-shaped elastic member forming the liquid-holding section is higher than that of the area of the inner pouch-shaped elastic member forming the liquid-holding section.

Also, an endoscope according to the present invention is characterized in that the endoscope includes: one of the above-described means for sustained release of liquid according to the present invention; a first infusion-exhaust means which can infuse or exhaust liquid or gas for inflating the inner pouch-shaped elastic member; and a second infusion-exhaust means which communicates with the liquid-infusing section and which can infuse or exhaust liquid.

Also, an instrument for endoscopic surgery according to the present invention includes: one of the above-described means for sustained release of liquid according to the present invention; a first infusion-exhaust means which can infuse or exhaust liquid or gas for inflating the inner pouch-shaped elastic member; and a second infusion-exhaust means which communicates with the liquid-infusing section and which can infuse or exhaust liquid.

### Advantageous Effects of Invention

According to the present invention, it is possible to obtain a user-friendly means for sustained release of liquid: making it possible to continue to stably and efficiently supply liquid like medical solution to also a diseased region that exists on a luminal wall surface and against gravity; making it possible to easily change a position of the means for sustained release of liquid after administration of liquid; making it possible to optionally regulate an amount of administration of liquid or a rate at which the liquid is supplied; and making it possible to supply plural kinds of liquid in turn, and it is possible to obtain an endoscope having the same and an instrument for endoscopic surgery having the same.

### Brief Description of Drawings

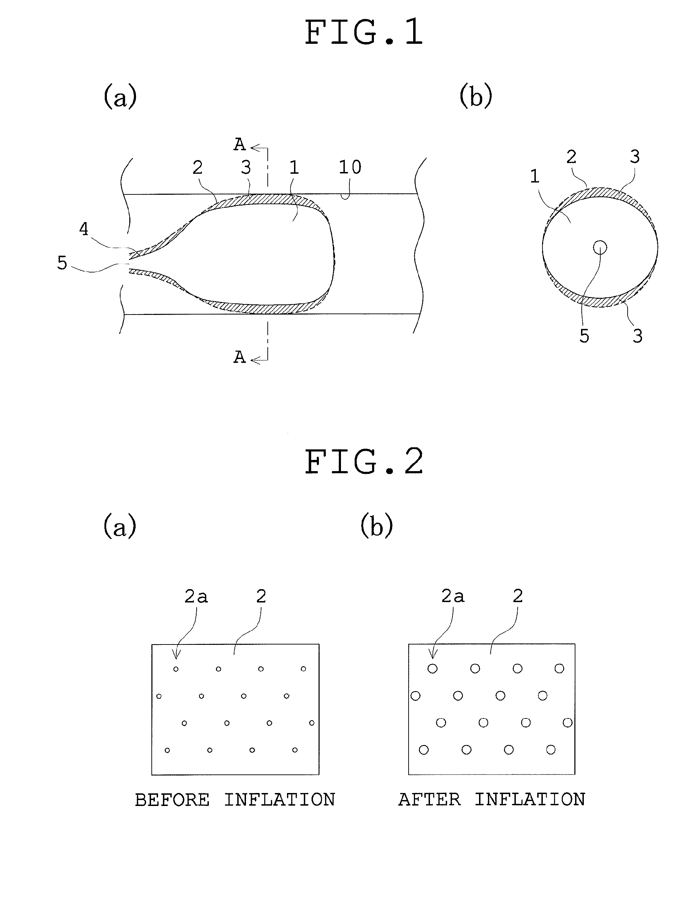

[Fig. 1] Fig. 1 is an explanatory view schematically showing a sketchy structure of a means for sustained release of liquid according to the first embodiment of the present invention, (a) of Fig. 1 is a cross-sectional view showing a state in which the inner pouch-shaped elastic member is inflated when a means for sustained release of liquid is viewed in the direction along the luminal wall, and (b) of Fig. 1 is a cross-sectional view taken along a line A-A in (a) of Fig. 1 when the means for sustained release of liquid is viewed in the direction of (a).
[Fig. 2] Fig. 2 is an explanatory view showing a surface structure of the outer pouch-shaped elastic member in the means for sustained release of liquid shown in Fig. 1, (a) of Fig. 2 is a view showing a state of the surface of the outer pouch-shaped elastic member before the outer pouch-shaped elastic member is inflated, and (b) of Fig. 2 is a view showing a state of the surface of the outer pouch-shaped elastic member after the outer pouch-shaped elastic member is inflated.

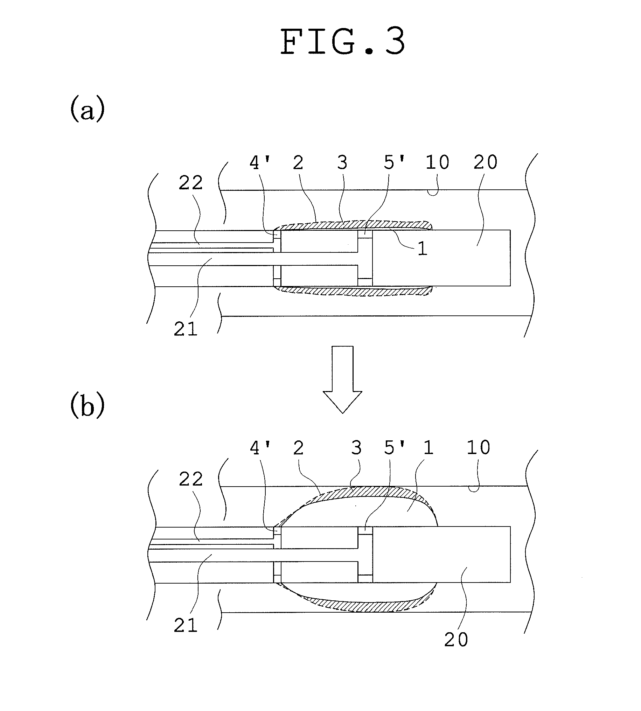

[Fig. 3] Fig. 3 is an explanatory view showing one configuration example in which the means for sustained release of liquid according to the first embodiment is integrated with the top end of an endoscope, (a) of Fig. 3 is a view showing a state of the means for sustained release of liquid before the inner pouch-shaped elastic member is inflated, and (b) of Fig. 3 is a view showing a state of the means for sustained release of liquid after the inner pouch-shaped elastic member is inflated.

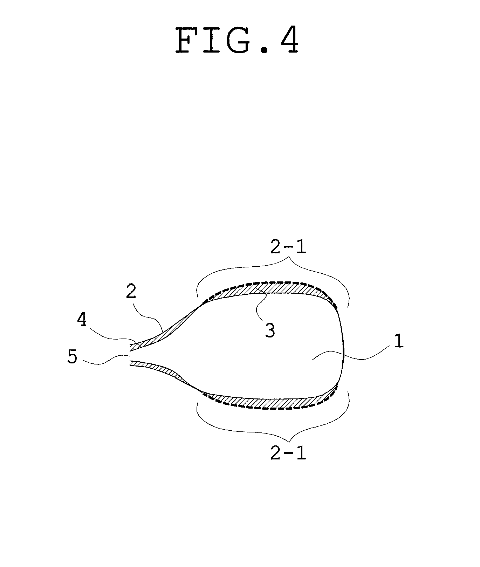

[Fig. 4] Fig. 4 is an explanatory view showing a primary part of a means for sustained release of liquid according to the second embodiment of the present invention.

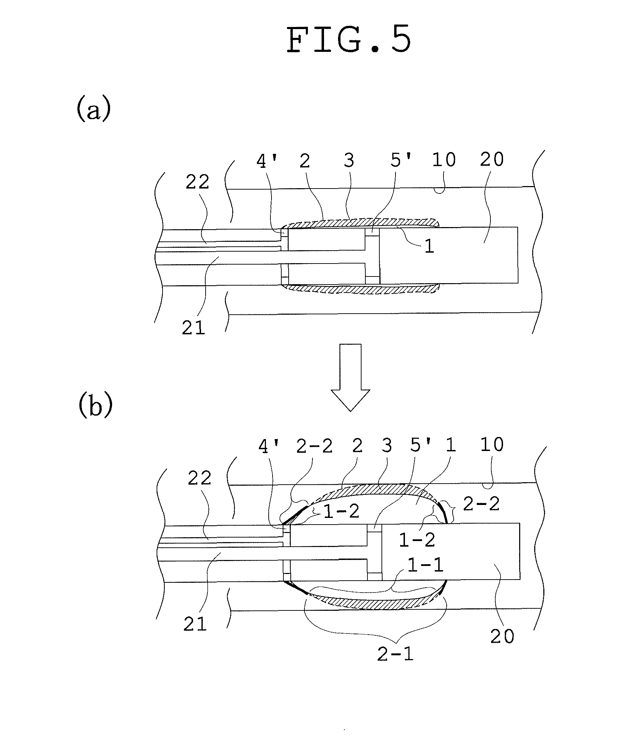

[Fig. 5] Fig. 5 is an explanatory view showing one configuration example in which a means for sustained release of liquid according to the third embodiment of the present invention is integrated with the top end of an endoscope, (a) of Fig. 5 is a view showing a state of the means for sustained release of liquid before the inner pouch-shaped elastic member is inflated, and (b) of Fig. 5 is a view showing a state of the means for sustained release of liquid after the inner pouch-shaped elastic member is inflated.
[Fig. 6] Fig. 6 is a perspective view showing one example of shape of the stent body of the drug sustained-release stent disclosed in Patent Literature 1.
[Fig. 7] Fig. 7 is a cross-sectional view showing one example of agent-applying instrument disclosed in Patent Literature 2.

### Mode for Embodying the Invention

Prior to the explanation of embodiments of the present invention, operation effects of the present invention are explained.

As in the present invention, a means for sustained release of liquid according to the present invention includes: an outer pouch-shaped elastic member; an inner pouch-shaped elastic member placed inside the outer pouch-shaped elastic member and capable of being inflated; a liquid-holding section holding liquid between the inner pouch-shaped elastic member and the outer pouch-shaped elastic member; and a minute hole provided for the outer pouch-shaped elastic member and capable of changing a diameter of the minute hole by inflation and deflation. As a result, when air or the like is infused into the inner pouch-shaped elastic member so as to inflate the inner pouch-shaped elastic member, pressure is applied to the outer pouch-shaped elastic member holding liquid with the liquid-holding section to inflate the outer pouch-shaped elastic member, so that the outer pouch-shaped elastic member presses against an target region for sustained release (like a diseased region on a luminal wall surface for example) to come into contact with the target region while the surface of the outer pouch-shaped elastic member is being elastically deformed, a diameter of the minute hole is expanded, and liquid held by the liquid-holding section passes through the minute hole to be sustainedly released from the surface of the outer pouch-shaped elastic member to the target region for sustained release on the outside. Also, when air or the like in the inner pouch-shaped elastic member is exhausted so as to deflate the inner pouch-shaped elastic member, the surface of the outer pouch-shaped elastic member separates from the target region for sustained release.

Accordingly, the above-described manner of configuring a means for sustained release of liquid according to the present invention makes it possible to optionally adjust an amount of administration and an administration rate with an amount of administration of liquid kept to the minimum by adjusting an amount of air or the like infused into the inner pouch-shaped elastic member.

Also, such a manner of configuring a means for sustained release of liquid according to the present invention makes it possible to easily move or retrieve the means by deflating the inner pouch-shaped elastic member after administration of liquid, so that it is possible to escape an obstacle to diagnosis of or treatment of the target region to which the liquid is supplied.

Also, such a manner of configuring a means for sustained release of liquid according to the present invention makes it possible to expand a diameter of the minute hole to sustainedly release liquid held by the liquid-holding section via the minute hole while the surface of the outer pouch-shaped elastic member is pressing on an target region for sustained release of the liquid with the surface of the outer pouch-shaped elastic member elastically deformed when the inner pouch-shaped elastic member is inflated, so that it is possible to continue to stably and efficiently supply the liquid like medical solution also to the target region for sustained release of liquid which exists against gravity.

Also, in a means for sustained release of liquid according to the present invention, a diameter of the minute hole of the outer pouch-shaped elastic member does not expand when the inner pouch-shaped elastic member is not inflated. As a result, it is possible to prevent liquid held by the liquid-holding section from leaking to the outside, so that it is possible to prevent loss of the liquid before the inner pouch-shaped elastic member is inflated.

Also, such a manner of configuring a means for sustained release of liquid according to the present invention makes it possible to release the outer pouch-shaped elastic member from pressure by exhausting air or the like from the inner pouch-shaped elastic member to deflate inner pouch-shaped elastic member. As a result, liquid in the liquid-holding section can be easily replaced with another liquid, so that plural kinds of liquid can be administered in turn by inflating the inner pouch-shaped elastic member again after replacement of liquid.

Also, when a means for sustained release of liquid according to the present invention further includes a liquid-infusing section capable of infusing liquid into the liquid-holding section between the inner pouch-shaped elastic member and the outer pouch-shaped elastic member, such a configuration of the means for sustained release of liquid makes it possible to achieve continuous sustained release of one kind of liquid or makes it possible to sustainedly release plural kinds of liquid by infusing a second liquid equal to or different from a first liquid in kind into the liquid-holding section between the outer pouch-shaped elastic member and the inner pouch-shaped elastic member from the liquid-infusion section after sustained release of the first liquid is complete.

Also, when the minute hole is provided for only a partial area of the surface of the outer pouch-shaped elastic member in a means for sustained release of liquid according to the present invention, it is possible to achieve sustained release of medical solution in such a way that medical solution is intensively supplied to a specific region.

Also, in a means for sustained release of liquid according to the present invention, when the partial area of the outer pouch-shaped elastic member is an area which forms the liquid-holding section in the outer pouch-shaped elastic member, it is possible to achieve sustained release of liquid in such a way that the medical solution is efficiently supplied to a desired region.

Also, in a means for sustained release of liquid according to the present invention, the outer pouch-shaped elastic member is configured in such a way that the elasticity of areas of the outer pouch-shaped elastic member excluding the area of the outer pouch-shaped elastic member forming the liquid-holding section is lower than that of the area of the outer pouch-shaped elastic member forming the liquid-holding section. As a result, the rest of areas of the outer pouch-shaped elastic member except the area forming the liquid-holding section in the outer pouch-shaped elastic member closely comes into contact with the rest of areas of the inner pouch-shaped elastic member except an area forming the liquid-holding section in the inner pouch-shaped elastic member when the inner pouch-shaped elastic member is inflated, so that it becomes easy to achieve sustained release of liquid held by the liquid-holding section in such a way that the liquid is intensively supplied to a desired region without dispersion of the liquid to the liquid-infusing section or the like.

Also, in a means for sustained release of liquid according to the present invention, the inner pouch-shaped elastic member is configured in such a way that the elasticity of areas of the inner pouch-shaped elastic member excluding the area of the inner pouch-shaped elastic member forming the liquid-holding section is higher than that of the area of the inner pouch-shaped elastic member forming the liquid-holding section. As a result, the rest of the areas of the outer pouch-shaped elastic member except the area forming the liquid-holding section in the outer pouch-shaped elastic member yet more closely comes into contact with the rest of the areas of the inner pouch-shaped elastic member except the area forming the liquid-holding section in the inner pouch-shaped elastic member when the inner pouch-shaped elastic member is inflated, in cooperation with the above configuration in which the elasticity of the areas of the outer pouch-shaped elastic member excluding the area of the outer pouch-shaped elastic member forming the liquid-holding section is lower than that of the area of the outer forming the liquid-holding section in the outer pouch-shaped elastic member, so that it becomes easy to achieve sustained release of liquid held by the liquid-holding section in such a way that the liquid is yet more intensively supplied to a desired region with dispersion of the liquid to the liquid-infusing section or the like prevented yet more.

Also, when an endoscope or an instrument for endoscopic surgery includes: one of the above-described means for sustained release of liquid according to the present invention; a first infusion-exhaust means capable of infusing or exhausting liquid or gas for inflating the inner pouch-shaped elastic member; and a second infusion-exhaust means communicating with the liquid infusing section and capable of infusing or exhausting liquid as in the present invention, it is possible to achieve an endoscope or an instrument for endoscopic surgery having the effects of the above-described means for sustained release of liquid according to the present invention.

Embodiments of the present invention are explained using the drawings, below.

### First Embodiment

Fig. 1 is an explanatory view schematically showing a sketchy structure of a means for sustained release of liquid according to the first embodiment of the present invention, (a) of Fig. 1 is a cross-sectional view showing a state in which the inner pouch-shaped elastic member is inflated when the means for sustained release of liquid is viewed in the direction along the luminal wall, and (b) of Fig. 1 is a cross-sectional view taken along a line A-A in (a) of Fig. 1 when the means for sustained release of liquid is viewed in the direction of (a). Fig. 2 is an explanatory view showing a surface structure of the outer pouch-shaped elastic member in the means for sustained release of liquid shown in Fig. 1, (a) of Fig. 2 is a view showing a state of the surface of the outer pouch-shaped elastic member before the outer pouch-shaped elastic member is inflated, and (b) of Fig. 2 is a view showing a state of the surface of the outer pouch-shaped elastic member after the outer pouch-shaped elastic member is inflated.

A means for sustained release of liquid according to the present embodiment is configured to include: an outer balloon 2 used as the outer pouch-shaped elastic member; and an inner balloon 1 used as the inner pouch-shaped elastic member and capable of being inflated, the inner balloon 1 being placed inside the outer balloon 2 so that two layers of the balloons 1 and 2 are formed, as shown in Fig. 1(a) and Fig. 1(b). Besides, the numeral reference 5 in Fig. 1 denotes an inner balloon-inflating infusion section for infusing gas or liquid into the inner balloon, and the numeral reference 10 in Fig. 1 denotes a luminal wall surface.

The inner and outer balloons 1 and 2 includes a liquid-holding section 3 which holds liquid between the inner and outer balloons 1 and 2. The liquid-holding section 3 is formed by a predetermined area of the outside surface of the inner balloon 1 and a predetermined area of the inside surface of the outer balloon 2 adhering firmly to each other. Besides, the liquid-holding section 3 in the example shown in Fig. 1 is formed to be an area of the inner and outer balloons 1 and 2 which faces to the luminal wall surface 10 (or the upper lateral peripheral surface and the lower lateral peripheral surface), as shown in Fig. 1(b).

The whole of the outside surface of the inner balloon 1 is covered with the outer balloon 2. The outer balloon 2 includes a plurality of minute holes 2a which change their diameters by inflation of and deflation of the outer balloon 2, as shown in Fig. 2(a) and Fig. 2(b).

Also, a liquid-infusing section 4 through which liquid can be infused into the liquid-holding section 3 is further provided between the inner balloon 1 and the outer balloon 1.

Fig. 3 is an explanatory view showing one configuration example in which the means for sustained release of liquid according to the first embodiment is integrated with the top end of an endoscope, (a) of Fig. 3 is a view showing a state of the means for sustained release of liquid before the inner pouch-shaped elastic member is inflated, and (b) of Fig. 3 is a view showing a state of the means for sustained release of liquid after the inner pouch-shaped elastic member is inflated.

Fig. 3 shows an example of the means for sustained release of liquid according to the present embodiment which is integrated with an annular member like a top end of an endoscope in order to use the means for sustained release of liquid according to the present embodiment for a luminal wall.

An endoscope shown in Fig. 3 includes a means for sustained release of liquid placed on a top end 20 of the endoscope, a first infusion-exhaust means 21, and a second infusion-exhaust means 22.

The means for sustained release of liquid of the example shown in Fig. 3 is placed on the lateral peripheral surface of the top end 20 of the endoscope. And, a liquid-infusing section 4' is provided for a portion between the inner balloon 1 and the outer balloon 1, the portion between the inner balloon 1 and the outer balloon 1 connecting with the lateral surface of the top end 20 of the endoscope. Also, an inner balloon-inflating infusion section 5' is provided for the lateral peripheral surface of the top end 20 of the endoscope. The other constitutions for the means for sustained release of liquid of the example shown in Fig. 3 are approximately same as those for the means for sustained release of liquid which were explained using Figs. 1 and 2.

The first infusion-exhaust means 21 is configured to be an infusion pipe the one end of which communicates with the inner balloon-inflating infusion section 5' and the other end of which is provided with a pump infusing or exhausting liquid or gas for inflating the inner pouch-shaped elastic member, the pump being not shown in the drawings.

The second infusion-exhaust means 22 is configured to be an infusion pipe the one end of which communicates with the liquid-infusing section 4' and the other end of which is provided with a pump infusing or exhausting liquid, the pump being not shown in the drawings.

A procedure for sustained release of optical clearing agent to a diseased region on a luminal wall surface with the means for sustained release of liquid according to the first embodiment having such a configuration is explained below using the configuration in which the means for sustained release of liquid is integrated with the endoscope, as in Fig. 3. The means for sustained release of liquid is moved to a position of a diseased region on the luminal wall surface 10 while the inner balloon 1 is not being inflated. Next, optical clearing agent is infused from the liquid-infusing section 4' into the liquid-holding section 3 through the second infusion-exhaust means 22 (refer to (a) of Fig. 3). Because the diameters of the minute holes 2a are not extended at this point in time, it is possible to prevent the optical clearing agent from leaking to the outside. Next, gas (or liquid) is infused from the inner-inflating infusion section 5' into the inner balloon 1 through the first infusion-exhaust means 21 so that the inner balloon is inflated. As a result, pressure is applied to the outer balloon 2 that holds the optical clearing agent with the liquid-holding section 3, so that the outer balloon 2 is inflated and presses on a diseased region on the luminal wall surface 10 while the surface of the outer balloon 2 is being elastically deformed. And then, the diameters of the minute holes 2a are extended, so that the optical clearing agent held by the liquid-holding section 3 is sustainedly released from the surface of the outer balloon 2 to the diseased region on the luminal wall surface 10 on the outside through the minute holes 2a (refer to (b) of Fig. 3).

The optical clearing agent gradually permeates the inside of the diseased region on the luminal wall surface 10 by sustained release of the optical clearing agent from the surface of the outer balloon 2 to the diseased region on the luminal wall surface 10. On the other hand, body fluid soaks out of the inside of the diseased region to enter into the liquid-holding section 3 through the minute holes a on the surface of the outer balloon 2. And, considerable amount of body fluid mixes with the optical clearing agent in the liquid-holding section 3 after a set time has elapsed, and the mix of the body fluid and the optical clearing agent causes deterioration of the function of the optical clearing agent. Accordingly, in the case where sustained release of the optical clearing agent to the diseased region on the luminal wall surface 10 is continued in such a situation, the gas (or liquid) having been infused into the inner balloon 1 is exhausted from the inner balloon 1 through the inner balloon-inflating infusion section 5' and the first infusion-exhaust means 21 so that the inner balloon 1 is temporarily deflated, and then, the mixture of the body fluid and the optical clearing agent in the liquid-holding section 3 is exhausted from the liquid-holding section 3 through the liquid-infusing section 4' and the second infusion-exhaust means 22. Afterward, another amount of optical clearing agent is infused from the liquid-infusing section 4' into the liquid-holding section 3 in the same manner as described above (refer to (a) of Fig. 3), and then, gas (or liquid) is infused into the inner balloon 1 through the inner balloon-inflating infusion section 5' so that the inner balloon 1 is inflated. As a result, the outer balloon 2 is inflated again to press closely on the diseased region on the luminal wall surface 10 while the surface of the outer balloon 2 is being elastically deformed, and then the diameters of the minute holes 2a are extended and the optical clearing agent held by the liquid-holding section 3 is sustainedly released from the surface of the outer balloon 2 to the diseased region on the luminal wall surface 10 on the outside through the minute holes 2a (refer to (b) of Fig. 3). As described above, infusion of optical clearing agent into the liquid-holding section 3, sustained release of optical clearing agent from the surface of the outer balloon 2 through infusion of gas (or liquid) into the inner balloon 1, exhaust of gas (or liquid) from the inner balloon 1, and exhaust of the liquid held by the liquid-holding section 3 are repeated until the diseased region on the luminal wall surface 10 has desirable transparency. When the diseased region on the luminal wall surface 10 has desirable transparency, the sustained release of the optical clearing agent is complete.

After completion of the sustained release of the optical clearing agent, the gas (or liquid) having been inflated into the inner balloon 1 is exhausted from the inner balloon 1 through the inner balloon-inflating injection section 5' and the first infusion-exhaust means 21 so that the inner balloon 1 is deflated. As a result, the outer balloon 2 separates from the diseased region on the luminal wall surface 10 which is made transparent. Also, the liquid in the liquid-holding section 3 is exhausted from the liquid-holding section 3 through the liquid-infusing section 4' and the second infusion-exhaust means 22. Next, the means for sustained release of liquid is moved to a position which is away from the diseased region on the luminal wall surface 10. As a result, it is possible to perform diagnosis of or treatment of the transparent diseased region on the luminal wall surface 10.

In the case where the diseased region having been made transparent is returned to its original state in which the diseased region is opaque after the completion of the diagnosis of or the treatment of the transparent diseased region on the luminal wall surface 10, the means for sustained release of liquid is moved to the position of the diseased region on the luminal wall surface 10 with the inner balloon 1 deflated. Next, saline is infused into the liquid-holding section 3 through the liquid-infusing section 4' and the second infusion-exhaust means 22. Because the diameters of the minute holes 2a are not extended at this point in time, it is possible to prevent the saline from leaking to the outside. Next, gas (or liquid) is infused into the inner balloon 1 through the first infusion-exhaust means 21 and the inner balloon-inflating infusion means 5' so that the inner balloon is inflated. As a result, pressure is applied to the outer balloon 2 holding the saline with the liquid-holding section 3, so that the outer balloon 2 is inflated to press on the diseased region on the luminal wall surface 10 while the surface of the outer balloon 2 is being elastically deformed, and the diameters of the minute holes 2a are extended, so that the saline held by the liquid-holding section 3 is sustainedly released from the surface of the outer balloon 2 to the diseased region on the luminal wall surface 10 on the outside through the minute holes 2a.

The means for sustained release of liquid according to the first embodiment makes it possible to optionally adjust administration amount or administration rate with an amount of administration of liquid kept to the minimum by adjusting an amount of infusion of air or the like into the inner balloon 1.

Also, in the means for sustained release of liquid according to the first embodiment, it is possible to easily move or retrieve the means for sustained release of liquid by deflating the inner balloon 1 after administration of liquid, so that it is possible to escape an obstacle to diagnosis of or treatment of a region to which liquid is administered.

Also, the means for sustained release of liquid according to the first embodiment: includes the liquid-holding section 3 holding liquid between the inner balloon 1 and the outer balloon 2; and is configured to make it possible to sustainedly release liquid held by the liquid-holding section 3 through the minute holes 2a by inflating the inner balloon 1 to make the surface of the outer balloon 2 press on an target region for sustained release of liquid with the surface of the outer balloon 2 elastically deformed and to extend the diameters of the minute holes. As a result, it is possible to continue to stably and efficiently supply liquid like medical solution (optical clearing agent or saline) to also a diseased region on the luminal wall surface 10 which exists against gravity. Also, in the means for sustained release of liquid according to the first embodiment, the diameters of the minute holes 2a of the outer balloon 2 are not extended when the inner balloon 1 is not inflated. As a result, it is possible to prevent liquid held by the liquid-holding section 3 from leaking to the outside, and it is possible to prevent loss of liquid before the inner balloon 1 is inflated.

Also, in the means for sustained release of liquid according to the first embodiment, the outer balloon 2 is released from pressure by exhausting air or the like in the inner balloon 1 to deflate the inner balloon 1. As a result, it becomes easy to replace liquid in the liquid-holding section 3 with another liquid, and it is possible to administer plural kinds of liquid in turn by inflating the inner balloon 1 again after replacement of liquid with another one.

Also, the means for sustained release of liquid according to the first embodiment further includes a liquid-infusing section between the inner balloon 1 and the outer balloon 2, and liquid can be infused into the liquid-holding section 3 through the liquid-infusing section. As a result, it is possible to sustainedly release one kind of liquid continuously or to sustainedly release plural kinds of liquid by infusing the second liquid equal to or different from the first liquid in kind into the space between the inner balloon 1 and the outer balloon 2 through the liquid-infusing section 3 after completion of sustained release of the first liquid.

Also, the endoscope according to the first embodiment includes the means for sustained release of liquid according to the first embodiment, the first infusion-exhaust means 21 by which liquid or gas for inflating the inner balloon 1 can be infused or exhausted, and the second infusing-exhaust means 22 which communicates with the liquid-infusing section 4' and by which liquid can be infused or exhausted. As a result, it is possible to achieve an endoscope having the above-described effects of the means for sustained release of liquid according to the first embodiment.

### Second Embodiment

Fig. 4 is an explanatory view showing a primary part of a means for sustained release of liquid according to the second embodiment of the present invention.

In a means for sustained release of liquid according to the present embodiment, minute holes 2a for the outer balloon 2 are provided only on an area 2-1 of the outer balloon 2, the area 2-1 forming the liquid-holding section 3 in the outer balloon 2.

The other configurations for the means for sustained release of liquid according to the second embodiment are approximately the same as those for the means for sustained release of liquid according to the first embodiment shown in Fig. 1.

In the means for sustained release of liquid according to the second embodiment, when the inner balloon 1 is inflated to inflate the outer balloon 2, the liquid is sustainedly release intensively from the liquid-holding section 3. As a result, it is possible to efficiently perform sustained release of medical solution to a diseased region of interest by making the liquid-holding section 3 sited at a position at which the liquid-holding section 3 faces to the diseased region of interest.

Besides, in the means for sustained release of liquid according to the second embodiment, the minute holes 2a for the outer balloon 2 may be provided for only a part of the area 2-1 forming the liquid-holding section 3. In this case, it is possible to sustainedly release liquid intensively to a particular diseased region.

The other operation effects of the means for sustained release of liquid according to the second embodiment are approximately the same as those of the means for sustained release of liquid according to the first embodiment.

### Third Embodiment

Fig. 5 is an explanatory view showing one configuration example in which a means for sustained release of liquid according to the third embodiment of the present invention is integrated with the top end of an endoscope, (a) of Fig. 5 is a view showing a state of the means for sustained release of liquid before the inner pouch-shaped elastic member is inflated, and (b) of Fig. 5 is a view showing a state of the means for sustained release of liquid after the inner pouch-shaped elastic member is inflated.

In a means for sustained release of liquid according to the present embodiment, the outer balloon 2 is configured in such a way that the elasticity of areas 2-2 of the outer balloon 2 excluding an area 2-1 forming the liquid-holding section 3 in the outer balloon 2 is lower than that of the area 2-1 forming the liquid-holding section 3. In addition, the inner balloon 1 is configured in such a way that the elasticity of areas 1-2 of the inner balloon 1 excluding an area 1-1 forming the liquid-holding section 3 in the inner balloon 1 is higher than that of the area 1-1 forming the liquid-holding section 3.

The other constitutions for the means for sustained release of liquid according to the third embodiment and the endoscope provided with the means are approximately the same as those of the means for sustained release of liquid and the endoscope provided with the means according to the first and second embodiments.

In the means for sustained release of liquid and the endoscope provided with the same according to the third embodiment, the outer balloon 2 is configured in such a way that the elasticity of the areas 2-2 of the outer balloon 2 excluding the area 2-1 forming the liquid-holding section 3 in the outer balloon 2 is lower than that of the area 2-1 forming the liquid-holding section 3. As a result, when the inner balloon 1 is inflated, the areas 2-2 of the outer balloon 2 excluding the area 2-1 forming the liquid-holding section 3 in the outer balloon 2 closely come into contact with the areas 1-2 of the inner balloon 1 excluding the area 1-1 forming the liquid-holding section 3 in the inner balloon 1, so that it becomes easy to sustainedly release liquid held by the liquid-holding section 3 intensively to an target region for sustained release without dispersion of the liquid to another portion like the liquid-infusing section 4. In addition, in the means for sustained release of liquid and endoscope provided with the means according to the third embodiment, the inner balloon 1 is configured in such a way that the elasticity of the areas 1-2 of the inner balloon 1 excluding the area 1-1 forming the liquid-holding section 3 in the inner balloon 1 is higher than that of the area 1-1 forming the liquid-holding section 3. As a result, when the inner balloon 1 is inflated, the areas 2-2 of the outer balloon 2 excluding the area 2-1 forming the liquid-holding section 3 yet more closely come into contact with the areas 1-2 of the inner balloon 1 excluding the area 1-1 forming the liquid-holding section 3, so that it becomes easy to sustainedly release the liquid held by the liquid-holding section 3 yet more intensively to the target region for sustained release with dispersion of the liquid to another portion like the liquid-infusing section 4 prevented yet more.

Up to now, the embodiments for means for sustained release of liquid and endoscopes provided with the same according to the present invention have been explained. However, a means for sustained release of liquid and an endoscope provided with the same according to the present invention is not limited to the configurations for the above-described embodiments. For example, an inner pouch-shaped elastic member and an outer pouch-shaped elastic member in the present invention is not limited to the balloons, and any member having elasticity and capable of inflating into a pouched shape may be used as an inner pouch-shaped elastic balloon or an outer pouch-shaped elastic member. Also, the above-described embodiments are explained using examples of constitution in which a means for sustained release of liquid is integrated with the top end of an endoscope. However, apparatus provided with a means for sustained release of liquid in the present invention is not limited to the top end of endoscope, and a means for sustained release of liquid according to the present invention may be integrated with an instrument for endoscopic surgery like forceps which is used together with an endoscope.

### Industrial Applicability

A means for sustained release of liquid according to the present invention, an endoscope having the same, and an instrument for endoscopic surgery having the same are useful for every field in which a diseased region existing against gravity is given medical solution while the medical solution is being kept on the diseased region for a set period of time and has to be diagnosed or treated immediately after giving the medical solution to the diseased region.

### Reference Signs List

- 1: inner pouch-shaped elastic member (inner balloon)
- 1-1: area forming liquid-holding section in inner balloon
- 1-2: areas excluding area forming liquid-holding section in inner balloon
- 2: outer pouch-shaped elastic member (outer balloon)
- 2a: minute hole
- 2-1: area forming liquid-holding section in outer balloon
- 2-2: areas excluding area forming liquid-holding section in outer balloon
- 3: liquid-holding section
- 4, 4': liquid-infusing section
- 5, 5': inner balloon-inflating infusion section
- 10: luminal wall surface
- 20: inserting part of the top end of endoscope
- 21: the first infusion-exhaust means (infusion pipe provided with a pump)
- 22: the second infusion-exhaust means (infusion pipe provided with a pump)
- 51: stent body
- 61: elongated stick-shaped base component
- 62: gas passage
- 63: balloon
- 64: agent-holding component

## Claims

1. A means for sustained release of liquid, **characterized in that** the means comprises
an outer pouch-shaped elastic member,
an inner pouch-shaped elastic member placed inside the outer pouch-shaped elastic member and capable of being inflated,
a liquid-holding section holding liquid between the inner pouch-shaped elastic member and the outer pouch-shaped elastic member, and
a minute hole provided for the outer pouch-shaped elastic member, a diameter of the minute hole being changed by inflation and deflation.

2. A means for sustained release of liquid according to claim 1, **characterized in that** the means further includes a liquid-infusing section which can infuse liquid into the liquid-holding section between the inner pouch-shaped elastic member and the outer pouch-shaped elastic member.

3. A means for sustained release of liquid according to claim 1 or 2, **characterized in that** a group of minute holes is provided for only a partial area of the outer pouch-shaped elastic member.

4. A means for sustained release of liquid according to claim 3, **characterized in that** the partial area of the outer pouch-shaped elastic member is an area which forms the liquid-holding section in the outer pouch-shaped elastic member.

5. A means for sustained release of liquid according to one of claims 1 to 4, **characterized in that** the elasticity of an area of the outer pouch-shaped elastic member excluding an area of the outer pouch-shaped elastic member forming the liquid-holding section is lower than that of the area of the outer pouch-shaped elastic member forming the liquid-holding section.

6. A means for sustained release of liquid according to one of claims 1 to 5, **characterized in that** the elasticity of an area of the inner pouch-shaped elastic member excluding an area of the inner pouch-shaped elastic member forming the liquid-holding section is higher than that of the area of the inner pouch-shaped elastic member forming the liquid-holding section.

7. An endoscope according to the present invention, **characterized in that** the endoscope comprises
a means for sustained release of liquid according to one of claims 1 to 6 according to claim 2,
a first infusion-exhaust means capable of infusing or exhausting liquid or gas for inflating the inner pouch-shaped elastic member, and
a second infusion-exhaust means communicating with the liquid-infusing section and capable of infusing or exhausting liquid.

8. An instrument for endoscopic surgery, **characterized in that** the instrument comprises
a means for sustained release of liquid according to one of claims 1 to 6 according to claim 2,
a first infusion-exhaust means capable of infusing or exhausting liquid or gas for inflating the inner pouch-shaped elastic member, and
a second infusion-exhaust means communicating with the liquid-infusing section and capable of infusing or exhausting liquid.

## Amended claims

### Amended claims under Art. 19.1 PCT

**1.** After Amendment) A means for sustained release of liquid, **characterized in that** the means comprises an outer pouch-shaped elastic member,
an inner pouch-shaped elastic member placed inside the outer pouch-shaped elastic member and capable of being inflated,
a liquid-holding section holding liquid between the inner pouch-shaped elastic member and the outer pouch-shaped elastic member,
minute holes provided for the outer pouch-shaped elastic member, diameters of the minute holes being changed by inflation and deflation, and
a liquid-infusing section capable of infusing liquid into the liquid-holding section between the inner pouch-shaped elastic member and the outer pouch-shaped elastic member, and
**characterized in that** the group of the minute holes is provided for only a partial area of the outer pouch-shaped elastic member.

**2.** After Amendment) A means for sustained release of liquid according to claim 1, **characterized in that** the partial area of the outer pouch-shaped elastic member is an area which forms the liquid-holding section in the outer pouch-shaped elastic member.

**3.** After Amendment) A means for sustained release of liquid according to claim 1 or 2, **characterized in that** the elasticity of an area of the outer pouch-shaped elastic member excluding an area of the outer pouch-shaped elastic member forming the liquid-holding section is lower than that of the area of the outer pouch-shaped elastic member forming the liquid-holding section.

**4.** After Amendment) A means for sustained release of liquid according to one of claims 1 to 3, **characterized in that** the elasticity of an area of the inner pouch-shaped elastic member excluding an area of the inner pouch-shaped elastic member forming the liquid-holding section is higher than that of the area of the inner pouch-shaped elastic member forming the liquid-holding section.

**5.** After Amendment) An endoscope according to the present invention, **characterized in that** the endoscope comprises
a means for sustained release of liquid according to one of claims 1 to 4,
a first infusion-exhaust means capable of infusing or exhausting liquid or gas for inflating the inner pouch-shaped elastic member, and
a second infusion-exhaust means communicating with the liquid-infusing section and capable of infusing or exhausting liquid.

**6.** After Amendment) An instrument for endoscopic surgery, **characterized in that** the instrument comprises
a means for sustained release of liquid according to one of claims 1 to 4,
a first infusion-exhaust means capable of infusing or exhausting liquid or gas for inflating the inner pouch-shaped elastic member, and
a second infusion-exhaust means communicating with the liquid-infusing section and capable of infusing or exhausting liquid.

**7.** Delete)

**8.** Delete)
